# EUROPEAN PATENT APPLICATION

(11) **EP 4 687 101 A1**
(43) Date of publication of application: **04.02.2026**
(21) Application number: 24192687.2
(22) Date of filing: 02.08.2024
(51) Int. Cl.: G06T 7/33

(54) **DEVICE AND METHOD FOR REGISTERING A MODEL OF A BODY PART AND A REAL WORLD IMAGE OF THE BODY PART**

(71) Applicant: OLYMPUS WINTER & IBE GMBH, 22045 Hamburg (DE)
(72) Inventor: KRAMES, Lorena, 56075 Koblenz (DE); SUPPA, Per, 22083 Hamburg (DE)
(74) Representative: Seemann & Partner Patentanwälte mbB

(57) **Abstract**

A device 20 and method for registering a model 34 of a body part and a real world image 32 of the body part. The device 20 comprises: an input interface 21 configured to receive 3D data of the model 34 and 2D data of the real world image 32, a display unit 24 configured to visualize the model 34 and the body part on a 2D display 26, a user input device 22 configured to receive user input data and to communicate the user input data to the display unit 24, which is configured to operate a mouse pointer on the 2D display 26 based on the user input data. The device 20 is further enhanced by a registration unit 30 configured to receive user input data, which, for at least a first to third pair comprising a reference P point and a corresponding reference point P*, by application of the mouse pointer, indicates a registration between the reference point P in the real world image 32 and the corresponding reference point P* in the 3D model 34, an expanding unit 36, configured to expand the 2D data of the reference points P in the real world image 32 into synthesized 3D data by adding depth information, which is derived from the 3D data of the model 34 of the body part and/or the 2D data of the real world image 32, a transformation unit 38 configured to determine a registration matrix for registering the 3D data of the model 34 of the body part and the synthesized 3D data of the real world image 32 of the body part using the 3D coordinates of the first to third pair of reference points P, P*.

## Description

The invention relates to a device for registering a model of a body part and a real world image of the body part. The device comprises an input interface configured to receive 3D data of the model of the body part and 2D data of the real world image of the body part, a display unit configured to visualize the model and the body part on a 2D display of the display unit and a user input device configured to receive user input data and to communicate the user input data to the display unit, wherein the display unit is configured to operate a mouse pointer on the 2D display based on the user input data. Furthermore, the invention relates to a method of registering a model of a body part and a real world image of the body part, the method comprising the steps of: receiving 3D data of the model of the body part and visualizing the model on a 2D display, receiving 2D data of the real world image of the body part and visualizing the body part on the 2D display, receiving user input data from a user input device, which operates as a mouse pointer on the 2D display.

Registering a 3D model of a body part, for example of an inner organ, with a real word image can assist the surgeon during conduction of a surgical procedure. Generally, there is a need for intraoperative navigation, which is associated with a complex cognitive process. This particularly applies to minimally invasive surgery, for example laparoscopic surgery. Intraoperative navigation requires a combination of knowledge about the general anatomy of the target organ or body part, the patient-specific anatomy (that can be different in case of aberrant
anatomy) of the target organ and specific knowledge about the spatial relationship between anatomical structures within the target organ of the patient. While knowledge concerning the first two points can be generally acquired during the preoperative stage (e.g. by preoperative imaging, such as Computer Tomography (CT) and/or Magnetic Resonance Imaging (MRI)), the knowledge concerning the third point can only be acquired throughout the laparoscopic surgical intervention itself. Therefore, it is of utmost importance to meticulously dissect the tissue of the target organ to avoid any damage to unforeseen anatomical structures throughout the laparoscopic procedure.

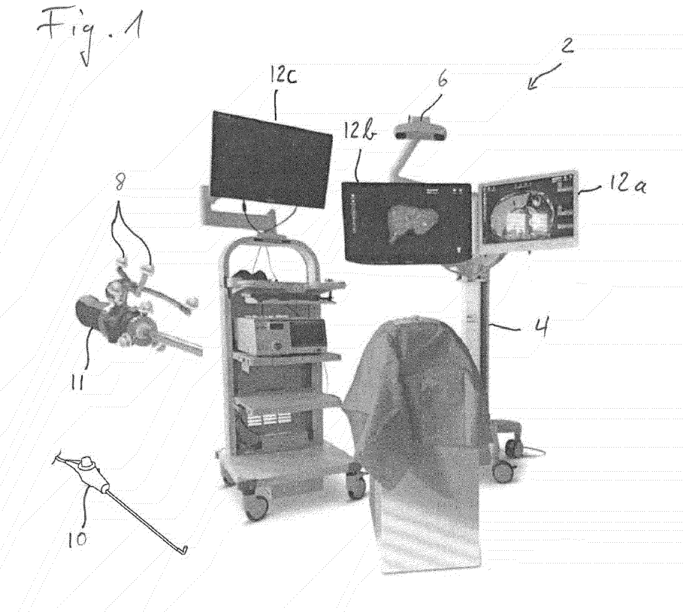

Figure 1 illustrates a classical minimally invasive surgery system 2, which is known from the prior art. The system 2 includes a navigation system 4 for supporting the surgeon with spatial anatomical information throughout the procedure. The navigation system 4 uses a hardware setup comprising an optical tracking camera 6, tracking markers 8 (e.g. reflective balls) that need to be attached to the surgical instrument 10 (partly shown) that should be tracked, and the laparoscopic camera 11, that may be a separate device as shown in Fig. 1, or that may be included in the surgical instrument 10. Moreover, there is a first monitor 12a showing the image of segmented 2D CT/MRI scans, a second monitor 12b that displays the reconstructed 3D model derived of an organ under treatment and a third monitor 12c, which is currently turned off and configured to show a laparoscopic camera image. The reconstructed 3D model is typically acquired during preoperative imaging. This device is not a part of the hardware setup.

In the conventional navigation system 4, the tracking camera 6 acquires the position of the tracking markers 8 and calculates the position and orientation of the surgical instrument 10 in the coordinate system of the navigation system 4.

The navigation system 4 is to provide additional information about spatial anatomy to the surgeon. This can be done by displaying the reconstructed 3D model derived from preoperative imaging modalities on the second monitor 12b and the intraoperative image as given by the laparoscopic camera on the third monitor 12c. Both, the intraoperative image and the 3D model, need to be aligned with each other to simplify the interpretation of the provided information. In technical terms, this step is called registration.

There is still a need for a robust and reliable solution to automatically perform the registration. It is inevitable to incorporate human knowledge into the registration process to achieve the desired result. To this end, the user is requested to select at least three points, better four points, on the intraoperative target organ. In the system depicted in figure 1, this can be done with the tip of the surgical instrument 10 that is tracked by the tracking camera 6. The tracking camera 6 determines the position and spatial orientation of the surgical instrument 10 in the coordinate system of the navigation system 4. After a calibration with regards to the surgical instrument 10, the system knows where the tip of the surgical instrument 10 is located in space. This makes it possible to acquire points in space.

Subsequently, the user is requested to select the three or four corresponding points on the preoperative 3D model, which is displayed on the second monitor 12b. Corresponding points can be used to estimate a transformation matrix that minimizes the difference between both point clouds (i.e., the point cloud consisting of the four points on the intraoperative target organ and the point cloud consisting of the four corresponding points on the preoperative 3D model). This transformation matrix can then be applied to the preoperative 3D model to change its pose in a way that it directly matches the pose of the target organ in the intraoperative image as given by the laparoscopic camera.

The system 2, which is illustrated in figure 1, requires a lot of hardware equipment. This equipment is space occupying, for example the three monitors 12a, 12b, 12c that are required to display the reconstructed 3D model and the real world image from the laparoscopic camera. This is a particular drawback within laparoscopic surgery, as the operating room (OR) is usually already equipped with a lot of technical equipment that limits the space required for the OR staff anyway. Moreover, the hardware is in particular designed for the navigation purpose and hence is associated with a significant amount of costs for such a system.

Furthermore, the navigation system 4 itself needs to be prepared for use prior to surgery. This mainly includes the calibration of the tracking camera 6 and the tracking markers 8 attached to the surgical instrument 10. As the tracking camera 6 can only track markers 8 that are within the line of sight of the tracking camera 6, it requires the tracking markers 8 on the surgical instrument 10 to be attached to a part of the device that stays outside of the patient's body (e.g. at the grip of the surgical devices). The same applies to a laparoscopic camera 11, if such a device is applied and no camera is included in the surgical instrument 10.

As the tip of the surgical instrument 10 is used to select or mark any point in the field of view of the optical camera providing the real world view on the target organ (i.e. correspondence selection), the position of the tracking markers 8 on the surgical instrument 10 in relationship to the tip of it need to be calibrated. To this end, another hardware device, namely a calibration unit, is used which needs to be especially manufactured for the surgical instruments that should be used.

This setup has several drawbacks as it requires to attach tracking markers 8 to the surgical instrument 10 and to the laparoscopic camera 11 (if applied) which might have a negative impact on the ergonomic design of the surgical instrument 10 and the laparoscopic camera 11 and hence has a disruptive effect on the surgeon's work. The tracking markers 8 themselves need to be attached to marker shields which in turn can be attached to the surgical devices. The marker shields are tailored for certain devices and hence are expensive. Moreover, the reflective tracking balls (tracking markers 8) are disposable and need to be exchanged after each procedure. They are expensive as well.

Furthermore, the calibration process assumes that the distance or a spatial relationship between the tracking markers 8 and the tip of the surgical instrument 10 always stays the same throughout the procedure. This might be a highly simplified assumption as, in particular, surgical instruments 10 are subject to bending effects, which are constantly changing the distance between tracking markers 8 and the tip of the instrument 10. These effects directly affect the accuracy of the registration of pre- and intraoperative images and thus introduce rather large registration errors.

Another drawback of the conventional system 2 is that the target organ (within the patient's body) needs to be within the field of view of the tracking camera 8 at any time throughout the procedure. Moreover, the provided registration result will only be accurate under the assumption, that the target organ would not change its shape or is subject to movement. However, both assumptions cannot be met as the patient is often turned around and brought in different positions during laparoscopic surgery and the target organ is subject to constant manipulation by the surgeon to facilitate improved access to different regions. In the former case, the calibration is not valid anymore and the whole calibration process needs to be repeated. In the latter case, the registration result is not valid anymore and re-registration based on corresponding point selection needs to be performed. Both of these steps are associated with a delay in the procedure.

It is an object of the invention to provide an enhanced device and method for registering a model of a body part and a real world image of the body part.

The object is solved by a device for registering a model of a body part and a real world image of the body part, the device comprising:
an input interface configured to receive 3D data of the model of the body part and 2D data of the real world image of the body part,
a display unit configured to visualize the model and the body part on a 2D display of the display unit,
a user input device configured to receive user input data and to communicate the user input data to the display unit, wherein the display unit is configured to operate a mouse pointer on the 2D display based on the user input data,
the device is further enhanced by
a registration unit configured to receive user input data, which, for at least a first to third pair comprising a reference point and a corresponding reference point, by application of the mouse pointer, indicates a registration between the reference point in the real world image and the corresponding reference point in the 3D model,
an expanding unit, configured to expand the 2D data of the reference points in the real world image into synthesized 3D data by adding depth information, which is derived from the 3D data of the model of the body part and/or the 2D data of the real world image,
a transformation unit configured to determine a registration matrix for registering the 3D data of the model of the body part and the synthesized 3D data of the real world image of the body part using the 3D coordinates of the first to third pair of reference points.

The user input device works in the same way as a computer mouse and can be used similar to a mouse pointer on the display unit. In comparison to conventional systems, the system according to aspects of the invention offers the advantage that both, the real world image and the 3D model of the body part are manipulated via the same software and the same user input device. The software runs on a processing device of the device for registering the model of the body part and the real world image thereof. There is no need for transfer or conversion of the coordinates of corresponding reference points from the real world coordinate system due to the coordinate system of the display. Furthermore, the system advantageously dispenses with expensive further hardware, like for example a tracking system. A conventional tracking system typically includes a tracking camera and tracking markers on the surgical instrument. Both units require installation space, need to be maintained and are cost-intensive. Advantageously, the registration process can be performed by using the single user input device. In particular, the user input device is a wireless device similar to 3D mouse. Because the system has reduced requirements with respect to hardware, it requires also less floorspace or installation space. This is particularly advantageous in an operating room environment. Summarizing, the system offers the following advantages: There is no need for an extensive hardware setup, except for the user input device. Furthermore, there is no extensive and time-consuming calibration procedure necessary before use. The system can dispense with a calibration process because there is no transfer of coordinates from the real world coordinate system to the coordinate system of the display. Advantageously, no modification of the surgical instruments like tracking balls or similar devices is necessary. These potentially negatively impact the ergonomic design of the surgical instruments. There is no necessity to work within a constant field of view of for example a tracking camera. Moreover, the operator/endoscopist does not need to look at a second monitor to enable the registration as everything will be displayed on the main monitor. Therefore, there is no need to lose focus on the main monitor.

In particular, the registration unit is configured to receive user input data, which, for at least a first to fourth pair comprising a reference point and a corresponding reference point, by application of the mouse pointer, indicates a registration between the reference point in the real world image and the corresponding reference point (P*) in the 3D model. The inclusion of a fourth pair of reference point and corresponding reference point improves the accuracy of the registration.

According to an advantageous embodiment of the invention, the device is further enhanced in that the display unit is configured to visualize the registered model and registered real world image in an overlay visualization, wherein in particular the 2D display is a single screen. Advantageously, the system dispenses with the requirement for a second screen. This is cost-saving and requires less installation space.

The device can be enhanced in that the expanding unit is further configured to perform the addition of the depth information to the 2D data of the at least one reference point in the real world image by assigning the depth information of the 3D data of the corresponding reference point of the model to the reference point of the real world image so as to provide the synthesized 3D data of the reference point. The real world image comprises a 2D information only. To perform the registration between the 2D real world image and the 3D model of the body part, it is necessary to convert the 2D image data of the real world image into data set of 3D data. In other words, it is necessary to expand the 2D reference points to become 3D reference points. By adding the spatial depth component of the 3D model to the 2D reference points of the real world image, the reference points can be transferred. This approach works under the assumption that the target body part as shown in the 2D real world image and the 3D model thereof are approximately in the same shape.

In a more sophisticated approach, according to an embodiment, the device is further enhanced in that the expanding unit is further configured to perform the addition of the depth information to the 2D data of the reference point in the real world image in that:
the expanding unit is further configured to perform a monocular depth estimation on the 2D data of the real world image and to assign the estimated depth values to the 2D coordinates of the 2D data of the real world image so as to provide synthesized 3D data of the real world image,
to assign the estimated depth information of the synthesized 3D data to the reference point so as to provide the synthesized 3D data of the reference point.

The monocular depth estimation (MDE) is an artificial intelligence-based task that estimates the depth coordinates of individual pixels in a 2D image from visual clues, such as objects and their relations to each other within the imaging frame.. For this purpose, a monocular depth estimation algorithm can be implemented in the expanding unit of the device. Areas or pixels of the body part in the real world image that are more far away from the camera plane tend to be displayed darker than areas or pixels in the real world image that are near to the camera plane. This effect can be enhanced by placing the light source near or beside the imaging device, for example in a tip of the laparoscopic camera or in a tip of the surgical instrument at which the camera is typically located.

In particular, the MDE analysis is performed for the complete 2D data set of the real world image, which means that the full 2D picture is assigned depth information.

According to still another advantageous embodiment of the invention, the device is further enhanced by a topological pattern analysis unit, which is configured to
perform a topological pattern analysis in a surrounding area of at least one of the reference points and the corresponding reference point(s) and
optimize the registration between the at least one reference point in the real world image and the corresponding reference point in the 3D model in that an optimized pair of reference points is selected, wherein the optimized reference point and the optimized corresponding reference point, are selected in that a best matching pattern analysis can be found in the surrounding area of the optimized pair of reference points.

The initially selected reference points can be regarded as starting points to trigger the optimization process. If the MDE was for example applied to estimate depth values for each individual pixel of the real world image, the topological analysis can select all points within the surrounding area to search for the best matching between the 3D coordinates of the 3D model and the expanded 3D data set of the real world image. The uniqueness of the reference points can therefore be described by evaluating the relationship of the selected reference points and the neighboring points in the surrounding area. This will significantly enhance the matching of the reference points, in particular in an application scenario, in which the real world image offers few textural information. The criterion of the best matching can be applied by using a threshold value. The similarity between the topological information in the data points of the 3D model and the expounded 3D data set of the real world image can be described by a parameter, for example a least square deviation. If this parameter is below certain threshold value, the similarity between the parts or areas can be considered as matching.

The embodiment can be further enhanced in that the topological pattern analysis unit is further configured to perform the pattern analysis on the 3D data of the model and the synthesized 3D data of the real world image, which is determined by performing the monocular depth estimation on the 2D data of the real world image and by assigning the estimated depth values to the 2D coordinates of the 2D data of the real world image so as to provide the synthesized 3D data of the real world image. In other words, the completed 2D data set of the real world image can be expanded into 3D data.

According to still another advantageous embodiment of the invention, the device further comprises an imaging unit, which is configured to determine the 3D data of the model of the body part during a preoperative imaging procedure, and further in particular comprising a surgical instrument having a camera, which is configured to acquire the 2D data of the real world image of the body part during a surgical procedure. This system provides an integrated solution for imaging and registering of acquired data.

Furthermore, the object is solved by a method of registering a model of a body part and a real world image of the body part, the method comprising the steps of:
receiving 3D data of the model of the body part and visualizing the model on a 2D display,
receiving 2D data of the real world image of the body part and visualizing the body part on the 2D display,
receiving user input data from a user input device, which operates as a mouse pointer on the 2D display,
characterized by
receiving, for at least a first to third pair, comprising a reference point and a corresponding reference point, user input data, which by application of the mouse pointer, indicates a registration between the reference point in the real world image and the corresponding reference point in the 3D model,
expanding the 2D data of the reference points in the real world image into synthesized 3D data by adding depth information, which is derived from the 3D data of the model of the body part and/or the 2D data of the real world image,
determining a registration matrix for registering the 3D data of the model of the body part and the synthesized 3D data of the real world image of the body part using the 3D coordinates of the first to third pair of reference points.

Same or similar advantages, which have been mentioned with respect to the device, apply to the method in the same or similar way and shall therefore not be repeated.

In particular, the method includes the step of receiving, for at least a first to fourth pair, comprising a reference point and a corresponding reference point, user input data, which by application of the mouse pointer, indicates a registration between the reference point in the real world image and the corresponding reference point in the 3D model. The inclusion of the fourth pair increases the accuracy of the registration.

Advantageously, the method further comprises the step of: visualizing the registered model and the registered real world image in an overlay visualization, wherein in particular, the overlay visualization is displayed on a single screen.

In an advantageous embodiment, the method further comprises the step of adding depth information to the 2D data of the at least one reference point in the real world image performed by assigning the depth information of the 3D data of the corresponding reference point of the same pair to the 2D data of the reference point so as to provide the synthesized 3D data of the reference point.

Furthermore, the method can advantageously be enhanced by the step of adding depth information to the 2D data of the reference point in the real world image further comprising the steps of:
performing monocular depth estimation on the 2D data of the real world image and assigning the estimated depth values to the 2D coordinates of the 2D data of the real world image so as to provide synthesized 3D data of the real world image,
assigning the estimated depth information of the synthesized 3D data to the reference point so as to provide the synthesized 3D data of the reference point.

In another advantageous embodiment of the invention, the method further comprises the steps of:
performing a topological pattern analysis in a surrounding area of at least one of the reference points and the corresponding reference point(s) and
optimizing the registration between the at least one reference point in the real world image and the corresponding reference point in the 3D model in that an optimized pair of reference points is selected, wherein the optimized reference point and the optimized corresponding reference point are selected in that a best matching pattern analysis can be found in the surrounding area of the optimized pair of reference points.

The method can be further enhanced in that the topological pattern analysis is performed on 3D data of the model and synthesized 3D data of the real world image, which is determined by performing monocular depth estimation on the 2D data of the real world image and by assigning the estimated depth values to the 2D coordinates of the 2D data of the real world image so as to provide the synthesized 3D data of the real world image.

In still another advantageous embodiment, the method is enhanced in that the 3D data of the model of the body part is determined during preoperative imaging and in particular the 2D data of the real world image of the body part is acquired by a surgical instrument during a surgical procedure. The surgical instrument is for example a surgical endoscope. The endoscope can have the rigid or flexible shaft. For example, the endoscope is a laparoscope.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a simplified perspective illustration of a conventional surgical system comprising the navigation system for the surgical instrument,
- Fig. 2: a simplified perspective illustration of a device for registering a model of a body part and a real world image of the body part,
- Fig. 3: a simplified schematic illustration of the device for registering,
- Fig. 4: a comparison between a real world image (Fig. 4a) and an overlay image (Fig. 4b), in which the real world image is depicted together with a representation of a 3D model of the respective body part,
- Fig. 5: a real world image (Fig. 5a) and the corresponding depth map (Fig. 5b), which is calculated by performing an MDE in the real world image,
- Fig. 6: a flowchart illustrating individual steps during performance of a method of registering a model of a body part and a real world image of the body part.

Fig. 1 is a simplified perspective illustration of the surgical system according to the prior art, which does not form part of the present invention.

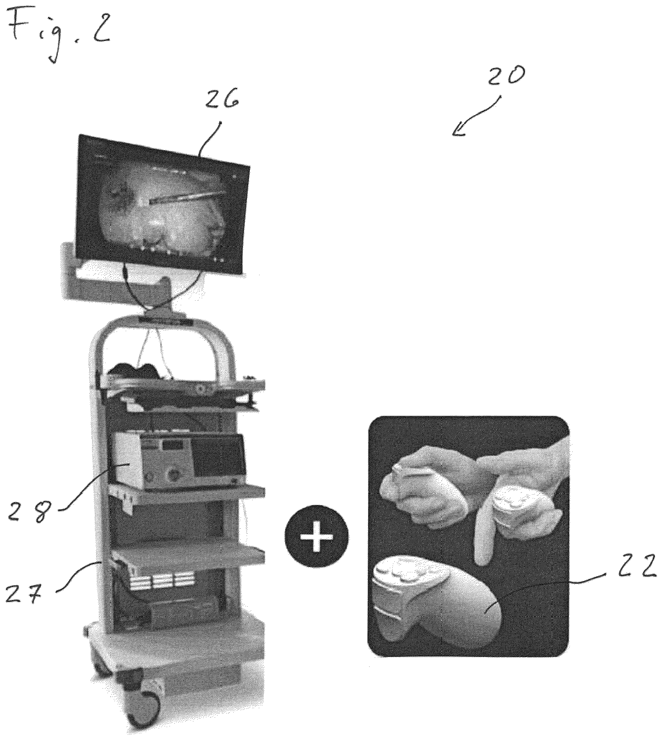

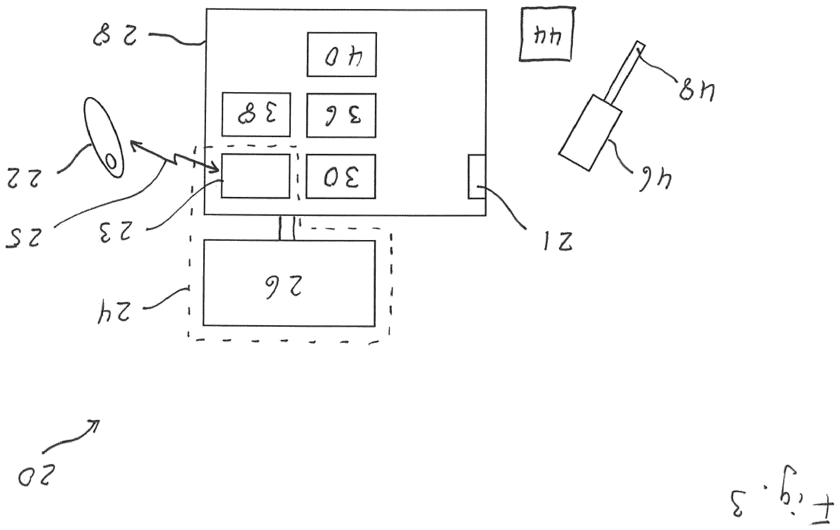

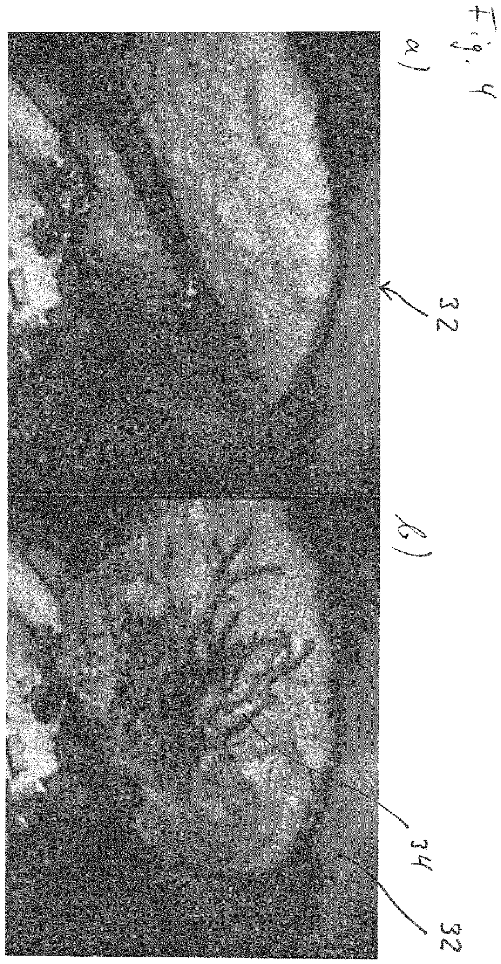

Fig. 2 is a simplified perspective illustration of a device 20 for registering of the model 34 (see Fig. 4) of the body part and a real world image 32 (see Fig. 4) of this body part. The body part can be in particular an inner organ of the patient. The device 20 comprises a main unit 28, which is installed on a rack 27. On top of the rack 27, there is a 2D display 26, which is for example a flatscreen display. Further details of the main unit 28 will be explained with reference to Fig. 3. The device 20 further comprises a user input device 22, which is in particular wirelessly connected to the main unit 28. The user input device 22 is configured to receive user input data. The operation of the user input device 22 is similar to a wireless 3D mouse. It is configured to operate a mouse pointer on the display 26, for example by tilting and twisting, wherein further actions or operations can be performed using the buttons on the user input device 22.

Fig. 3 schematically illustrates the functional units of the device 20. At the main unit 28, there is an input interface 21, which is configured to receive 3D data of the model 34 of the body part. This 3D data can for example be acquired using an imaging unit 44, which can form part of the device 20. The imaging unit 44 is for example a computer tomograph (CT) or a magnetic resonance imaging (MRI) unit. It can be used for inspection of an inner organ of the patient. The data of the imaging unit 44 is used to calculate the model 34 of the body part, for example of an inner organ of a patient. The 3D data of the model 34 may be communicated from the imaging unit 44 to the input interface 21 of the main unit 28. Alternatively, the 3D data of the model 34 may be communicated from another source, for example a computer configured to calculate the 3D Model. For example, the 3D data may be derived from acquiring a 2D CT/MRI scan, which is then transformed into a 3D model. Furthermore, the input interface 21 receives 2D data of a real world image 32 of the body part. The real world image 32 is acquired for example during surgery, which is conducted with the surgical instrument 46 on an inner organ of a patient, which was previously subject to inspection with the imaging unit 44. The surgical instrument 46 is for example a laparoscope. At the distal end of the shaft of the surgical instrument 46, there is a camera 48 for capturing the real world image. The data of the real world image is communicated via the input interface 21 to the main unit 28 of the device 20.

The main unit 28 of the device 20 further comprises a display unit 24, which is for example an image processor and is configured to visualize the model 34 and the body part on the 2D display 26. The model 34 and the body part can be shown on a single screen. For example, the output of the main unit 28 may be a two dimensional image showing the intraoperative video image and the 3D model in a certain orientation and at certain scale superimposed on the intraoperative video image.

The user input device 22 is configured to receive user input data, for example by manual manipulation thereof. The user input data is communicated to the display unit 24. This in turn is configured to operate a mouse pointer on the 2D display 26 based on the user input data. In other words, the user input device 22 functions as a mouse pointer on the 2D display 26.

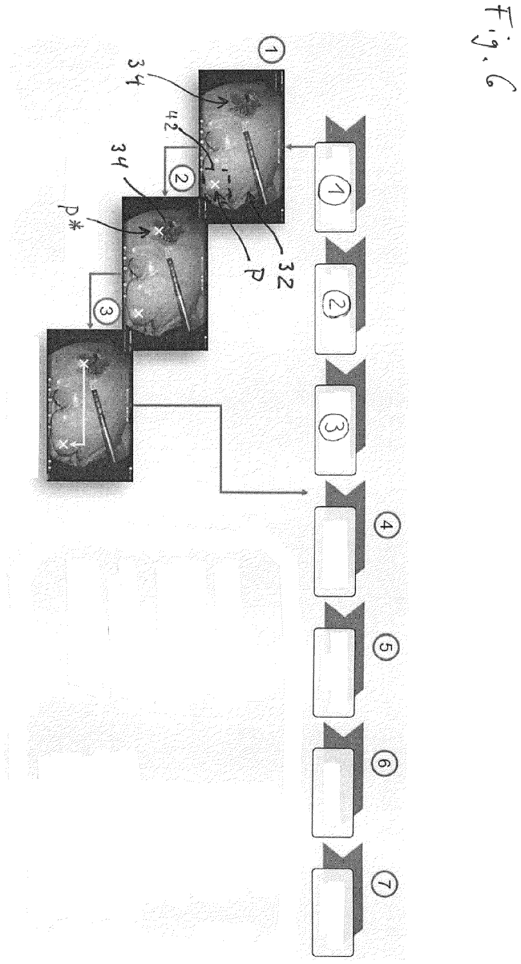

The main unit 28 further comprises a registration unit 30, which is also configured to receive user input data. This user input is indicative of at least a first to third pair of reference points P, P*. Each pair of reference points comprises a reference point P and a corresponding reference point P*. The reference points are selected on the real world image and on the model 34 by operation of the user input device 22 and respective selection on the 2D display 26. This selection indicates a registration between a reference point P in the real world image 32 and a corresponding reference point P* in the 3D model 34. Further details will be explained with reference to Fig. 6.

The data of the real world image 32 is 2D data. For registration with the 3D model 34, it is necessary to convert the 2D data into 3D data. For this purpose, there is an expanding unit 36 also forming part of the main unit 28. The expanding unit 36 is configured to expand the 2D data of the plurality of reference points P in the real world image 32 into synthesized 3D data. This is performed by adding depth information to the coordinates in the 2D data of the reference point P. This depth information is derived from the 3D data of the model 34 of the body part and/or the 2D data of the real world image 32. Details will be given further below.

The main unit 28 further comprises a transformation unit 38, which is configured to determine a registration matrix for registering the 3D data of the model 34 of the body part and the synthesized 3D data of the real world image 32 of the body part. The transformation matrix is calculated from the coordinates of the first to third reference point P and the corresponding reference point P*. The user selection of the reference point P and the corresponding reference point P* indicates that the reference points P in the real world image 32 and the corresponding reference points P* in the model correspond to each other, which means that the reference points P, P* point to the same position on the displayed body part. In other words, the reference point P, which is selected in the real world image 32 can be found at the position of the corresponding reference point P* in the model 34. Based on the coordinates of the four pairs of reference points P, P*, a transformation matrix for matching the real world image 32 and the model 34 to each other can be calculated for a respective pixel or point of the model 34 or the real world image 32, respectively.

As a result, an overlay image can be displayed on the 2D display 26 assisting the surgeon during surgery of the body part.

Fig. 4a illustrates a real world image 32, which is for example captured with the camera 48 at the tip of the surgical instrument 46. The real world image 32 shows an inner organ of a patient, by way of an example a part of the liver. Figure 4b shows an overlay image of the real world image 32 and the model 34 of the inner organ. The data of the model 34 is preoperatively acquired and shows for example a position and pathway of blood vessels or other inner structures of the organ. This information can assist the surgeon during conduction of surgery.

The determination of depth information, which is added to the 2D data of the real world image 32 to provide the synthetic 3D data can be performed by application of different approaches.

According to the first approach, the expanding unit 36 is configured to perform the addition of the depth information to the 2D data of the reference point P in the real world image 32 by simply assigning the depth information of the 3D data of the corresponding reference point P* of the model to the reference point P in the real world image 32.

According to another approach, the expanding unit 36 is further configured to perform the addition of the depth information to the 2D data of the reference point P in the real world image 32 as follows: The expanding unit 36 performs monocular depth estimation (MDE) on the 2D data of the real world image 32. Subsequently, the expanding unit 36 assigns the estimated depth values, which result from the monocular depth estimation, to the 2D data of the real world image 32 so as to provide the synthesized 3D data of the real world image 32. The assignment of the estimated depth information can be performed for the reference points P or for every pixel or point of the real world image 32.

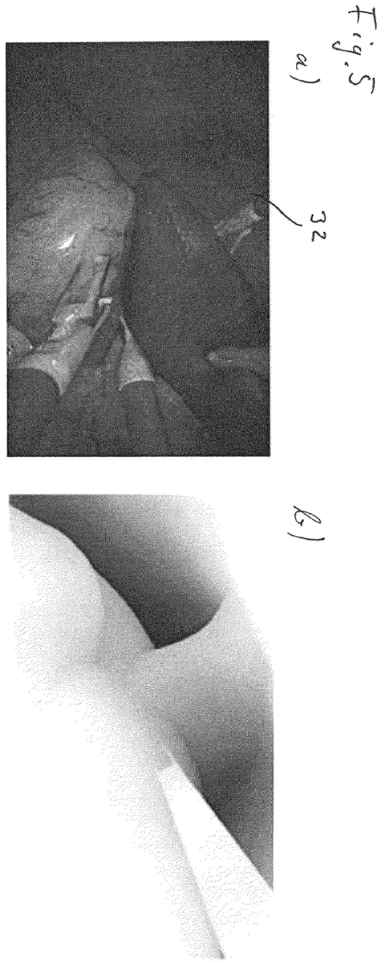

Fig. 5a is a real world image, which is for example acquired by the camera 48 of the surgical instrument 46. Fig. 5b illustrates the corresponding depth map in greyscale coding, which is calculated by performing monocular depth estimation on the real world image 32, which is shown in fig. 5a.

The device 20 in particular implements a software, which is known as AXR-7 available from the Applicant. The AXR-7software in its present configuration is a light-weight navigation system that allows the surgeon to perform laparoscopic procedures by having access to reconstructed 3D model data of the target organ and the intraoperative image of the target organ as given by the laparoscopic camera on the same monitor, i.e. on a 3D model 34 and on a real world image 32. The surgeon can manipulate the view on the 3D model 34 by changing its orientation and can translate the 3D model 34 on the 2D display 26 throughout the procedure to gain additional information regarding spatial anatomy within the target organ. Moreover, the surgeon can manually superimpose the 3D model 34 on the intraoperative target organ which mimics the registration process. This can be done by using a user input device 22. The device 20 implements an enhanced version of the AXR-7 software, which aims to introduce a semi-automatic registration functionality. This will be explained in the following.

The enhanced AXR-7 software is operated in combination with a mouse pointer feature that is controlled by the user input device 22. The enhanced AXR-7 software can include a function that allows the use of the user input device 22 for controlling of a virtual mouse pointer (VMP). The VMP works in the same way as a computer mouse (hardware) and can be used as a pointer (software) on the 2D display 26. The advantage of the AXR-7 software compared to conventional navigation systems is that both, the intraoperative real world image 32 of the target organ as a body part and the 3D representation, i.e. the 3D model 34 (in particular a preoperative 3D model) of the target organ are already visualized on the same operating room monitor and can be controlled via the same software (i.e., the AXR-7 software). Therefore, the transfer of corresponding reference points that were tracked in physical space into the software space is not required with this setup anymore.

The 3D model 34 can be displayed within a picture-in-picture (PiP) layer and superimposed on the laparoscopic video real world image 32. This is important, as it technically allows to separate the pixels as given by the 2D video image from the voxels (3D equivalent of a pixel) which are given by the PiP layer.

The user can now use the VMP to select at least four reference points P on the target organ as shown in the 2D intraoperative video real world image 32. Subsequently, the user selects the four corresponding reference points on the preoperative 3D model 34 of the target organ using the VMP. However, as the selected reference points P on the target organ in the video image are only 2D points (i.e. points that are described by x- and y-coordinates), they need to be expanded to become 3D points. To add a spatial depth component (i.e. z-coordinate) to the 2D points, the z-coordinate as given by the corresponding point on the 3D model 34 can be transferred to the 2D reference point P. This approach works under the assumption that the target organ as shown in the 2D intraoperative real world video image 32, and the target organ as given by the 3D model 34 are approximately in the same shape (i.e. no deformation). A more advanced approach to transform the 2D points into 3D points is given by the use of monocular depth estimation (MDE). MDE can be artificial intelligence-based. It aims to estimate the depth values of individual pixels in a 2D real world image 32 and therefore allows to transform 2D points into 3D points. The result of this expansion is a point cloud.

Another advantage of the device 20 is that the initially selected reference points P, P* correspondences can be regarded as starting points to trigger an optimization process. For instance, if MDE was applied to estimate depth values for each individual pixel in the intraoperative 2D real world video image 32, the software can use all points within the real world image 32 to search for the most unique points within this image. The uniqueness of a point can be described, for example, by evaluating the relationship of the selected point with its neighboring points. This is a topological pattern analysis that aims to identify the most unique points or the most unique cluster of points. The same process can then be applied on the 3D model 34 using the selected corresponding reference point P* as initial starting point to search for an enhanced reference point P* (or a cluster of points) that matches the reference point P (or cluster of points) on the 2D real world video image even better. With this approach, inaccurate user-based selection of corresponding point pairs P, P* can be compensated with an initial automatic optimization process to finally obtain more accurate registration results.

Fig. 6 illustrates a flowchart of individual steps that are performed during execution of the method of registering the 3D model 34 of the body part and the real world image 32 of the body part.

In step 1., the virtual mouse pointer, by operation of the user input device 22, is applied to select the reference point P on the 2D real world video image 32. The real world image 32 is displayed on the 2D display 26 by together with the 3D model 34. In step 2., The virtual mouse pointer is used to select the corresponding reference point P*in the 3D model 34. In step 3., The depth value of the selected corresponding reference point P* is transferred to the reference point P in the real world image 32. As an alternative, the depth information can be derived from MDE. Step 4. illustrates is the repetition of this process for three more pairs of reference points P, P*. In step 5., The transformation matrix is calculated from the coordinates of the reference points P and the corresponding reference points P*. The registration is applied in step 6. In step 7., The registered real world image 32 and 3D model 34 are displayed on the 2D screen 26. Steps 3. to 7. are performed fully automatically. Only the steps 1. and 2., which means the selection of the reference point P in the real world image 32 and the selection of the corresponding reference point P* in the 3D model 34, have to be performed manually.

In the drawings, the same or similar types of elements or respectively corresponding parts are provided with the same reference numbers in order to prevent the item from needing to be reintroduced.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 2: surgical system
- 4: navigation system
- 6: tracking camera
- 8: tracking markers
- 10: surgical instrument
- 11: laparoscopic camera
- 12a: first monitor
- 12b: second monitor
- 12c: third monitor
- 20: device
- 21: input interface
- 22: user input device
- 23: display processing unit
- 24: display unit
- 25: wireless datalink
- 26: 2D display
- 27: rack
- 28: main unit
- 30: registration unit
- 32: real world image
- 34: 3D model
- 36: expanding unit
- 38: transformation unit
- 40: topological pattern analysis unit
- 42: surrounding area
- 44: imaging unit
- 46: surgical instrument
- 48: camera
- P: reference point
- P*: corresponding reference point

## Claims

1. A device (20) for registering a model (34) of a body part and a real world image (32) of the body part, the device (20) comprising:
an input interface (21) configured to receive 3D data of the model (34) of the body part and 2D data of the real world image (32) of the body part,
a display unit (24) configured to visualize the model (34) and the body part on a 2D display (26) of the display unit (24),
a user input device (22) configured to receive user input data and to communicate the user input data to the display unit (24), wherein the display unit (24) is configured to operate a mouse pointer on the 2D display (26) based on the user input data, **characterized by**
a registration unit (30) configured to receive user input data, which, for at least a first to third pair comprising a reference (P) point and a corresponding reference point (P*), by application of the mouse pointer, indicates a registration between the reference point (P) in the real world image (32) and the corresponding reference point (P*) in the 3D model (34),
an expanding unit (36), configured to expand the 2D data of the reference points (P) in the real world image (32) into synthesized 3D data by adding depth information, which is derived from the 3D data of the model (34) of the body part and/or the 2D data of the real world image (32),
a transformation unit (38) configured to determine a registration matrix for registering the 3D data of the model (34) of the body part and the synthesized 3D data of the real world image (32) of the body part using the 3D coordinates of the first to third pair of reference points (P, P*).

2. The device (20) according to claim 1, wherein the display unit (23) is configured to visualize the registered model (34) and registered real world image (32) in an overlay visualization, wherein in particular the 2D-display (26) is a single screen.

3. The device (20) of claim 1 or 2, wherein the expanding unit (36) is further configured to perform the addition of the depth information to the 2D data of the at least one reference point (P) in the real world image (32) by assigning the depth information of the 3D data of the corresponding reference point (P*) of the model (34) to the reference point (P) of the real world image (32) so as to provide the synthesized 3D data of the reference point (P).

4. The device (20) of claim 1 or 2, wherein the expanding unit (36) is further configured to perform the addition of the depth information to the 2D data of the reference point (P) in the real world image (32) in that:
the expanding unit (36) is further configured to perform a monocular depth estimation on the 2D data of the real world image (32) and to assign the estimated depth values to the 2D coordinates of the 2D data of the real world image (32) so as to provide synthesized 3D data of the real world image (32),
to assign the estimated depth information of the synthesized 3D data to the reference point (P) so as to provide the synthesized 3D data of the reference point (P).

5. The device (20) of one of the preceding claims, further comprising a topological pattern analysis unit (40) is, which is configured to
perform a topological pattern analysis in a surrounding area (40) of at least one of the reference points (P) and the corresponding reference point(s) (P*) and
optimize the registration between the at least one reference point (P) in the real world image (32) and the corresponding reference point (P*) in the 3D model (34) in that an optimized pair of reference points (P, P*) is selected, wherein the optimized reference point and the optimized corresponding reference point, are selected in that a best matching pattern analysis can be found in the surrounding area (42) of the optimized pair of reference points.

6. The device (20) of claim 5, wherein the topological pattern analysis unit (40) is further configured to perform the pattern analysis on the 3D data of the model (34) and the synthesized 3D data of the real world image (32), which is determined by performing the monocular depth estimation on the 2D data of the real world image (32) and by assigning the estimated depth values to the 2D coordinates of the 2D data of the real world image (32) so as to provide the synthesized 3D data of the real world image (32).

7. The device (20) of one of the preceding claims, further comprising an imaging unit (44), which is configured to determine the 3D data of the model (34) of the body part during a preoperative imaging procedure, and further in particular comprising a surgical instrument (46) having a camera (48), which is configured to acquire the 2D data of the real world image (32) of the body part during a surgical procedure.

8. A method of registering a model (34) of a body part and a real world image (32) of the body part, the method comprising the steps of:
receiving 3D data of the model (34) of the body part and visualizing the model (34) on a 2D display (26),
receiving 2D data of the real world image (32) of the body part and visualizing the body part on the 2D display (26),
receiving user input data from a user input device (22), which operates as a mouse pointer on the 2D display (26),
**characterized by**
receiving, for at least a first to third pair, comprising a reference point (P) and a corresponding reference point (P*), user input data, which by application of the mouse pointer, indicates a registration between the reference point (P) in the real world image (32) and the corresponding reference point (P*) in the 3D model (34),
expanding the 2D data of the reference points (P) in the real world image (32) into synthesized 3D data by adding depth information, which is derived from the 3D data of the model (34) of the body part and/or the 2D data of the real world image (32),
determining a registration matrix for registering the 3D data of the model (34) of the body part and the synthesized 3D data of the real world image (32) of the body part using the 3D coordinates of the first to third pair of reference points (P, P*).

9. The method of claim 8, further comprising the step of: visualizing the registered model (34) and the registered real world image (32) in an overlay visualization, wherein in particular, the overlay visualization is displayed on a single screen.

10. The method of claim 8 or 9, wherein the step of adding depth information to the 2D data of the at least one reference point (P) in the real world image (32) is performed by assigning the depth information of the 3D data of the corresponding reference point (P*) of the same pair to the 2D data of the reference point (P) so as to provide the synthesized 3D data of the reference point (P).

11. The method of any of claims 8 to 10, wherein the step of adding depth information to the 2D data of the reference point (P) in the real world image (32) further comprises the steps of:
performing monocular depth estimation on the 2D data of the real world image (32) and assigning the estimated depth values to the 2D coordinates of the 2D data of the real world image (32) so as to provide synthesized 3D data of the real world image (32),
assigning the estimated depth information of the synthesized 3D data to the reference point (P) so as to provide the synthesized 3D data of the reference point (P).

12. The method of any of claims 8 to 11, further comprising the steps of:
performing a topological pattern analysis in a surrounding area (34) of at least one of the reference points (P) and the corresponding reference point(s) (P*) and
optimizing the registration between the at least one reference point (P) in the real world image (32) and the corresponding reference point (P*) in the 3D model (34) in that an optimized pair of reference points is selected, wherein the optimized reference point and the optimized corresponding reference point are selected in that a best matching pattern analysis can be found in the surrounding area (42) of the optimized pair of reference points.

13. The method of claim 12, wherein the topological pattern analysis is performed on the 3D data of the model (34) and synthesized 3D data of the real world image (32), which is determined by performing monocular depth estimation on the 2D data of the real world image (32) and by assigning the estimated depth values to the 2D coordinates of the 2D data of the real world image (32) so as to provide the synthesized 3D data of the real world image (32).

14. The method of any of claims 8 to 13, wherein the 3D data of the model (34) of the body part is determined during preoperative imaging and in particular the 2D data of the real world image (32) of the body part is acquired by a surgical instrument (46) during a surgical procedure.
